# EUROPEAN PATENT SPECIFICATION

(11) **EP 0 250 953 B1**
(45) Date of publication and mention of the grant of the patent: **27.01.1993**
(21) Application number: 87108336.6
(22) Date of filing: 10.06.1987
(51) Int. Cl.: B01D 11/02

(54) **Process for the extraction of oily fruits**
Extraktionsverfahren für ölhaltige Früchte
Procédé d'extraction des fruits oléagineux

(30) Priority: 17.06.1986 IT 2081386
(43) Date of publication of application: 07.01.1988
(73) Proprietor: INDENA S.p.A., 20141 Milano (IT)
(72) Inventor: Bombardelli, Ezio, I-20141 Milano (IT); Pozzi, Roberto, I-20141 Milano (IT); Bertani, Marco, I-20141 Milano (IT)
(74) Representative: Huber, Bernhard, Dipl.-Chem.

(56) References cited:
- EP-A- 0 065 222
- BE-A- 1 000 036
- DE-A- 3 133 032
- DE-A- 3 319 184
- FR-A- 2 480 754
- KIRK-OTHMER:"Encyclopedia of Chemical Technology", 3rd ed., 1984, Supplement volume "Supercritical Fluids": "Advantages of SFE" pp.876-877, "Environmental considerations" p.886, "Food and pharmaceutical applications of SFE" pp. 887-888
- Gazzetta Chimica Italiana, 118, 1988, pp.823-826
- Journal of Medical Plant Research, 1980, vol. 40, pp.262-270
- Gaz. med. de France 89, no. 17, p. 2041, 1982

## Description

The present invention refers to a new process of extraction of Serenoa repens fruits and to the extract obtained by this process.

The fruits of Serenoa repens, a small palm tree growing in the United States, in Northern Africa and in Spain, have been used for a long time in the preparation of alcoholic, hydroalcoholic and oily extracts, that are used in the treatment of pathological conditions connected with prostatic adenoma. The use of Serenoa repens in this therapy has been known for a long time (see, for example, "Dispensatory of the United States of America", 1888); also known are the extraction of the drug with apolar solvents and, in part, the qualitative and quantitative composition of the oil that can be obtained in this way.

Recently (FR-A-2 480 754) a process for the preparation of Sabal serrulatum (also known as Serenoa repens) extracts, by extraction with apolar solvents, petroleum ether or halogenated hydrocarbons, in the presence of antioxidant substances and in an inert gas atmosphere,has been described. In this process, the addition to the extraction medium of antioxidant substances and the use of inert gases in the main industrial steps for the preparation of the final products are essential in order to prevent the oxidation of fatty acids and insaturated alcohols that the extract contain in a high percentage. The obtained extract must be subsequently treated with coal to eliminate undesired substances, and finally dried.

For obtaining extracts to be used in the pharmaceutical field the use of CO₂ under hypercritical conditions was known (Kirk Othmer "Encyclopedia of Chemical Technology", 1984, pages 876-877 and 886-888).

The use of CO₂ in hypercritical conditions has been already proposed for the drying of plant extracts (EP-A-65222) and for the removal of allergens from Arnica montana extracts (DE-A-33 19 184). DE-A-31 33 032 discloses a process and an apparatus for extraction of different materials, including plant materials, with gases in hypercritical conditions.

Now it has been surprisingly found, and this is the object of the invention, that the oily extract of Serenoa repens can be advantageously prepared at industrial level by extraction from the fruits with CO₂ in hypercritical conditions, as specified below, in a selective way and without having to add, in the extraction step, natural or synthetic additives with a stabilizing function, the solvent itself preventing the oxidation by a perfect de-aeration of the medium. The extract obtained in this way, moreover, requires no decoloration.

In comparison with the processes known so far, the process according to the invention has the substantial advantages to allow a perfect extraction of the unaltered lipidic material, yielding, after a complete evaporation of the solvent, and filtration on a dehydrating agent, an extract devoid of residual solvents or non-volatile contaminating agents, that are generally present in the solvents used in industry; moreover, this extract can be directly used in the most common pharmaceutical formulations, without further purifications. The obtained products may optionally be submitted to drying under vacuum, at temperatures ranging from 35° to 80°C.

In the process of the present invention, the extraction is carried out according to claim 2 and an extract according to claim 1 is obtained. The evaporation is preferably carried out at temperatures ranging from 20°to 30°C, at pressure ranging from 50 to 70 bars.

The process of the invention can be carried out in a continuous cycle.

After drying the products, a steady yellow orange, dehydrated and deodorized oil is obtained, being the most volatile part eliminated in the process.

The chemico-physical characteristics of the obtained products are the following:
. density: about 0.9;
. refraction index: about 1.45 at 20°C;
. content in substances that cannot be saponified: about 3%.

This substance that cannot be saponified consists mainly of a mixtures of phytosterols, the most important of which is β-sitosterol, and of a mixture of saturated C₂₀-C₃₀ alcohols, the most important of which is octacosanol.

In normal conditions the content in β-sitosterol ranges from 0.2 to 0.35%, whereas n-octacosanol ranges from 0.1 to 0.2%.

In the oil, these alcohols and sterols are present in the form of esters, and in a negligible quantity in the free form.

The quantitative determination of these alcohols and sterols can be easily carried out by gas-chromatography, after methanolysis and sylilation of capillary column, according to methods described in the literature (Martinelli et al., Sixth International Symposium on Capillary Chromatography, Riva del Garda, May 14, 1985).

The oil obtained in this way, showed, during biochemical and pharmacological tests, a significant anti-androgenic, hormone-regulating and anti-oedematous action, in agreement with what reported in the literature for similar products, prepared according to less convenient methods.

The examples reported below illustrate the invention without limiting the scope thereof.

### EXAMPLE 1 (comparative example)

In a 5 l extractor equipped with heating and with all the accessories for pressure control, 1.2 kg of Serenoa repens fruits, finely ground by a cryocontusion process (cold grinding at -20°C), were extracted.

The drug was extracted, using subsequently 10 l of continuous recycling CO₂, for 2 hours, at a temperature of 35° and 250 bar. After evaporation of the solvent, the extracted material was recovered and dried at 2 mm/Hg and 45°C for 24 hours.

In this way, 0.138 kg of a yellow orange clear oil, with the following characteristics, were obtained:
- Density: 0.896
- Refraction index: 1.46
- Substances that cannot be saponified: 2.52%
- Saponification index: 230
- Content in free fatty acids: 86%.

### EXAMPLE 2

In a 5 l extractor, equipped with heating and with all the accessories for pressurization and its controls, 1.2 kg of Serenoa Repens fruits finally ground by cryogrinding were extracted as in Example 1.

The drug was on the whole extracted with 10 l of continuous recycling CO₂ for 2 hours at a temperature of 45°C and 220 bar, keeping a temperature of 25°C and 50 bar in the condenser.

When the extraction was complete, after a complete evaporation of CO₂ from the condenser, the extracted oil saturated with water was recovered and filtered on 12 g of anhydrous sodium sulphate.

145 g of yellow orange oil were obtained; the oil was dried under vacuum at 2 mm/Hg at 50° for a complete dehydration, and it has the same characteristics as the oil obtained in Example 1.

## Claims

1. Extract of Serenoa repens fruits obtained by an extraction in which CO₂ in hypercritical conditions is used as solvent at a temperature of 45°C and a pressure of 220 bar.

2. A process for the extraction of Serenoa repens fruits characterized in that CO₂ in hypercritical conditions is used as solvent at a temperature of 45°C and a pressure of 220 bar.

3. A process according to patent claim 2 characterized in that CO₂ is evaporated, after the extraction step, at temperatures ranging from 20 to 30°C, at pressures ranging from 50 to 70 bar.

4. A process according to patent claim 2 or 3 characterized in that the obtained extract is subsequently submitted to dehydration.

## Patentansprüche

1. Extrakt von Serenoa repens-Früchten, erhalten durch eine Extraktion, worin CO₂ unter hyperkritischen Bedingungen als Lösungsmittel bei einer Temperatur von 45°C und bei einem Druck von 220 bar verwendet wird.

2. Verfahren zur Extraktion von Serenoa repens-Früchten, dadurch gekennzeichnet, daß CO₂ unter hyperkritischen Bedingungen als Lösungsmittel bei einer Temperatur von 45°C und bei einem Druck von 220 bar verwendet wird.

3. Verfahren gemäß Anspruch 2, dadurch gekennzeichnet, daß CO₂ nach der Extraktionsstufe bei Temperaturen im Bereich von 20 bis 30°C und bei Drucken im Bereich von 50 bis 70 bar verdampft wird.

4. Verfahren gemäß Anspruch 2 oder 3, dadurch gekennzeichnet, daß der erhaltene Extrakt anschließend einer Dehydratation unterzogen wird.

## Revendications

1. Extrait de fruits de serenoa repens obtenu par extraction dans laquelle on utilise du CO₂ comme solvant dans des conditions surcritiques à une température de 45°C et une pression de 220 bars.

2. Procédé pour l'extraction de fruits de serenoa repens caractérisé en ce qu'on utilise du CO₂ comme solvant dans des conditions surcritiques à une température de 45°C et une pression de 220 bars.

3. Procédé selon la revendication 2, caractérisé en ce que le CO₂ est mis en évaporation après l'étape d'extraction à une température allant de 20°C à 30°C et à des pressions allant de 50 à 70 bars.

4. Procédé selon la revendication 2 ou 3, caractérisé en ce que l'extrait obtenu est ensuite soumis à la déshydratation.
